# FASCICULE DE BREVET EUROPEEN

(11) **EP 2 440 144 B1**
(45) Date de publication et mention de la délivrance du brevet: **02.04.2014**
(21) Numéro de dépôt: 10734157.0
(22) Date de dépôt: 11.06.2010
(51) Int. Cl.: A61B 17/70

(54) **DISPOSITIF DE PROTECTION DES ÉTAGES ADJACENTS D'UN SEGMENT RACHIDIEN**
VORRICHTUNG FÜR DEN SCHUTZ VON ANGRENZENDEN EBENEN EINES SPINALEN SEGMENTS
DEVICE FOR PROTECTING ADJACENT LEVELS OF A SPINAL SEGMENT

(30) Priorité: 11.06.2009 FR 0902829
(43) Date de publication de la demande: 18.04.2012
(73) Titulaire: Clariance, 62000 Dainville (FR)
(72) Inventeur: TORNIER, Alain, F-38330 Saint-Ismier (FR)
(74) Mandataire: Vuillermoz, Bruno
(86) Numéro de dépôt international: PCT/FR2010/000427
(87) Numéro de publication internationale: WO 2010/142874

(56) Documents cités:
- WO-A-2007/052975
- US-A1- 2008 281 361

## Description

La présente invention est relative à un dispositif de protection des étages adjacents d'un segment rachidien d'une colonne vertébrale, instrumenté par un dispositif d'ostéosynthèse.

Dans le cas d'une fusion de plusieurs vertèbres, on constate que la capacité de mobilité de ces dernières est plus ou moins supprimée. Cette perte de mobilité dépend principalement du nombre de vertèbres immobilisées par le dispositif d'ostéosynthèse rachidien. Du fait de cette perte de mobilité, le patient recherchera à compenser en sur-sollicitant les étages adjacents.

En ce qui concerne la partie lombaire de la colonne vertébrale qui est très mobile comparée à la partie thoracique, la recherche de mobilité se fera préférentiellement sur les étages sus-jacents ou supérieurs, du fait du bras de levier important que représente le tronc.

On constate que cette recherche de mobilité se focalise sur un seul étage vertébral et principalement sur celui se trouvant immédiatement au-dessus du segment rachidien instrumenté, conduisant à moyen terme à une détérioration dudit étage vertébral.

On connaît des dispositifs de protection des étages adjacents d'un segment rachidien instrumenté qui sont constitués par exemple de tiges flexibles, articulées ou autres, fixées par l'intermédiaire de vis pédiculaires ancrées dans la vertèbre de l'étage sus-jacent.

Le document WO 2007052975 A1 décrit un dispositif selon le préambule de la revendication 1.

On note que ces dispositifs de protection ne sont ni plus ni moins qu'une extension de la fixation postérieure réalisée par le dispositif d'ostéosynthèse rachidien bloquant de manière quasi définitive l'étage sus-jacent à protéger.

Le dispositif de protection suivant la présente invention a pour objet de permettre que le patient, à la recherche d'une mobilité supplémentaire sur les étages sus-jacents d'un segment rachidien instrumenté puisse le faire en rééduquant plusieurs vertèbres et non uniquement celle se trouvant juste au-dessus, en limitant les amplitudes notamment en flexion - extension qui sont les principaux mouvements au niveau de la partie lombaire de la colonne vertébrale.

Le dispositif de protection suivant la présente invention vient en complément du dispositif d'ostéosynthèse rachidien permettant la fusion du segment rachidien instrumenté afin de réaliser une contention limitant l'extension de la flexion de la vertèbre sus-jacente.

Le dispositif de protection suivant la présente invention permet de réaliser une contention non traumatisante pour les tissus environnants. Le dispositif de protection suivant la présente invention peut être également utilisé en cas d'une ablation des épineuses des étages vertébraux du segment rachidien instrumenté.

Le dispositif de protection suivant la présente invention comprend des tiges de liaison constituées de connecteurs immobilisés en translation et en rotation sur la tige de liaison correspondante, d'une cale de soutien dans laquelle est logée l'épineuse Ve de la vertèbre sus-jacente Vc se trouvant juste au-dessus du segment rachidien Sr instrumenté et des ligaments, supérieur et inférieur, permettant de réunir d'une part l'épineuse Ve de la vertèbre sus-jacente Vc à la cale de soutien et d'autre part la cale de soutien aux connecteurs.

Le dispositif de protection suivant la présente invention comporte pour chaque connecteur un logement latéral à profil interne cylindrique ouvert vers l'extérieur pour permettre l'encliquetage et l'introduction de la tige de liaison correspondante, des moyens de serrage permettant l'immobilisation en translation et en rotation de chaque connecteur sur la tige de liaison correspondante, des rainures coopérant avec des moyens de blocage pour la retenue des ligaments supérieurs et inférieurs.

Le dispositif de protection suivant la présente invention comporte pour chaque connecteur au niveau de chaque sortie des rainures une empreinte destinée à recevoir la cale de soutien.

Le dispositif de protection suivant la présente invention comporte des rainures qui débouchent à l'intérieur d'une cavité ménagée au centre du connecteur et à l'intérieur de laquelle coopère, suivant une direction perpendiculaire à celle desdites rainures, une came permettant le serrage des ligaments supérieurs et inférieurs.

Le dispositif de protection suivant la présente invention comporte une cale de soutien qui est réalisée dans un matériau élastique de manière à pouvoir se déformer sous une pression extérieure et plus particulièrement sous la pression de la vertèbre sus-jacente Vc se trouvant juste au-dessus du segment rachidien Sr instrumenté.

Le dispositif de protection suivant la présente invention comporte une cale de soutien qui présente un profil en forme de U renversé comprenant un pont supérieur se prolongeant par des jambes de soutien dans lesquelles sont ménagées des rainures pour permettre respectivement la mise en place et la retenue des ligaments, supérieur et inférieur.

Le dispositif de protection suivant la présente invention comprend une première rainure qui est ouverte vers l'extérieur et ménagée sur le côté de chaque jambe de soutien de la cale de soutien.

Le dispositif de protection suivant la présente invention comprend une seconde rainure qui est ouverte vers l'extérieur et ménagée de manière continue dans l'épaisseur de chaque jambe de soutien et du pont supérieur de la cale de soutien.

Le dispositif de protection suivant la présente invention comprend un pont supérieur qui comporte un logement permettant la mise en place de l'épineuse Ve de la vertèbre sus-jacente Vc.

Le dispositif de protection la présente invention comprend une rainure qui passe en dessous du logement ménagé dans le pont.

Le dispositif de protection suivant la présente invention comprend une seconde rainure comportant une ouverture vers l'extérieur réalisée suivant une direction qui est perpendiculaire à celle prévue pour la première rainure.

Le dispositif de protection suivant la présente invention comprend un connecteur qui comporte un logement latéral à profil interne cylindrique permettant l'encliquetage et l'introduction de la tige de liaison correspondante, un alésage fileté débouchant à l'intérieur dudit logement et coopérant avec une vis de serrage permettant l'immobilisation en translation et en rotation de chaque élément de fixation, une rainure de profil oblong traversant le connecteur et permettant le passage du ligament synthétique, une cavité ménagée au centre du connecteur et à l'intérieur de laquelle débouche suivant une direction perpendiculaire à celle de la rainure un alésage fileté permettant d'une part l'introduction d'une clef de verrouillage à l'intérieur de ladite cavité et d'autre par le serrage d'un contre-écrou permettant d'immobiliser en rotation ladite clef de verrouillage.

Le dispositif de protection suivant la présente invention comprend une clef de verrouillage qui est constituée d'un doigt cylindrique comportant d'une part une tête pourvue d'une empreinte et d'autre part à l'opposé de ladite tête et suivant un axe vertical et central une rainure séparant ledit doigt cylindrique en deux parties distinctes, ladite rainure étant prévue pour être traversée par le ligament synthétique lorsque la clef de verrouillage est placée à l'intérieur de la cavité du connecteur.

Le dispositif de protection suivant la présente invention comprend un contre écrou qui comporte un alésage central et traversant pour le passage d'un outil afin que ce dernier puisse venir se loger à l'intérieur de l'empreinte de la clef de verrouillage en vue d'entraîner en rotation ladite clef de verrouillage à l'intérieur de la cavité du connecteur correspondant pour l'enroulement du ligament autour du doigt cylindrique.

Le dispositif de protection suivant la présente invention comprend une cale de soutien qui présente un profil en forme de U renversé comprenant un pont supérieur relié par deux jambes de soutien agencées de manière à passer de part et d'autre de l'épineuse Ve de la vertèbre Vb du segment rachidien Sr instrumenté, ledit pond supérieur comportant un logement coopérant avec l'épineuse Ve de la vertèbre sus-jacente Vc, tandis que lesdites jambes de soutien comportent d'une part sur le profil externe du U renversé une rainure ouverte vers l'extérieur et permettant la mise en place, la retenue et le guidage du ligament synthétique et d'autre part à chaque extrémité libre un pied se prolongeant par une collerette pourvue d'une gorge périphérique permettant la retenue de la cale de soutien à l'intérieur d'un logement de forme complémentaire réalisé à l'intérieur de chaque connecteur.

Le dispositif de protection suivant la présente invention comprend une rainure qui traverse chaque pied et la collerette afin que cette dernière se trouve lors du montage de la cale dans les connecteurs dans le prolongement de la rainure.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en perspective illustrant le dispositif de protection suivant la présente invention monté en complément par exemple sur un dispositif d'ostéosynthèse rachidien à tige de liaison connu en soi permettant la fusion d'un segment rachidien d'une colonne vertébrale.
Figure 2 est une vue en perspective éclatée montrant les différents éléments constituant le dispositif de protection et le dispositif d'ostéosynthèse rachidien suivant la présente invention.
Figure 3 est une vue en perspective représentant le dispositif de protection suivant la présenté invention assemblé sur un dispositif d'ostéosynthèse rachidien connu en soi.
Figure 4 est une vue en perspective éclaté illustrant les différents éléments constituant le dispositif de protection suivant la présente invention.
Figure 5 est une vue en perspective montrant une variante du dispositif de protection suivant la présente invention.
Figure 6 est une vue en perspective éclatée montrant les différents éléments constituant le dispositif de protection suivant la figure 5 et le dispositif d'ostéosynthèse rachidien suivant la présente invention.
Figure 7 est une vue en perspective représentant le dispositif de protection de figure 5 assemblé sur un dispositif d'ostéosynthèse rachidien connu en soi.
Figures 8 et 9 sont des vues en perspective éclatée illustrant les différents éléments constituant le dispositif de protection de figure 5 suivant la présente invention.

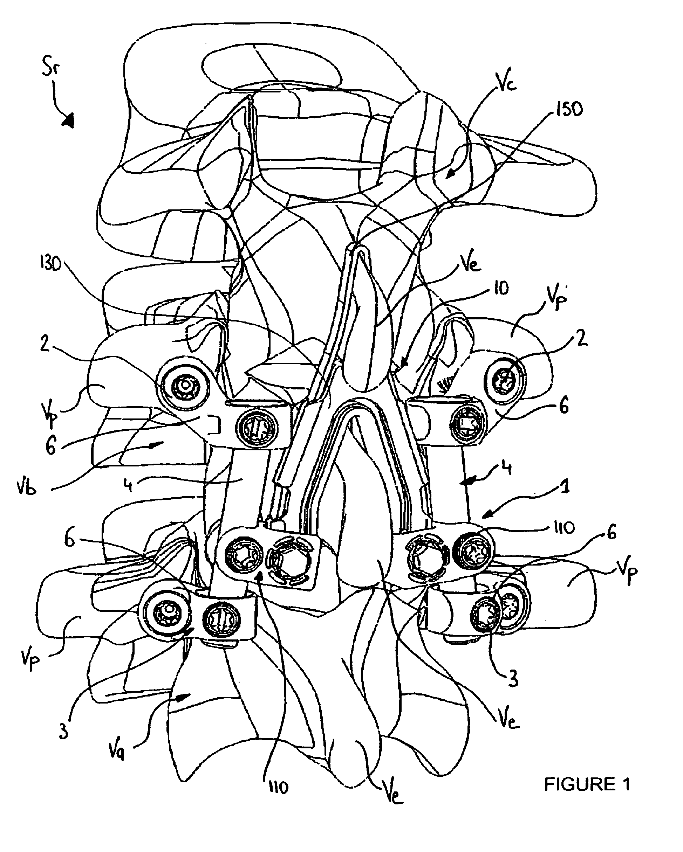

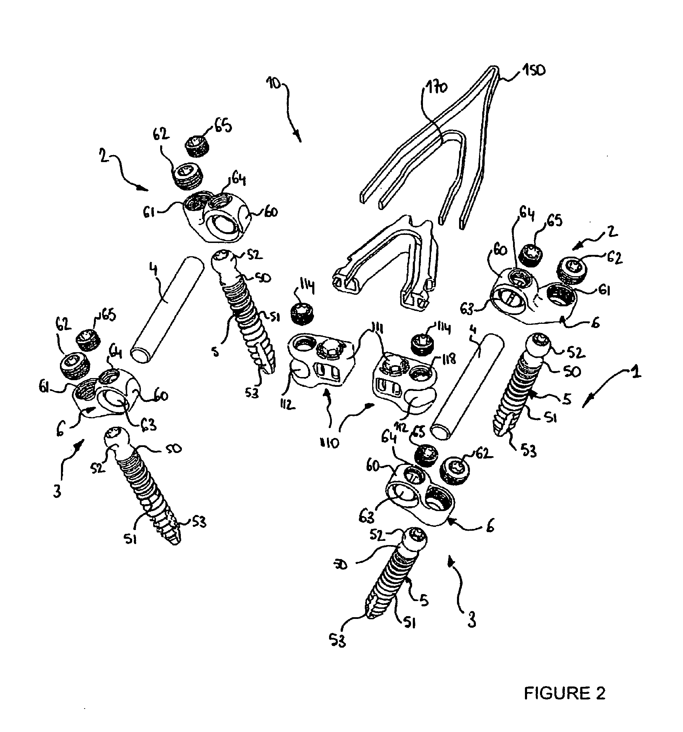

On a représenté en figures 1 et 2 un segment rachidien instrumenté Sr d'une colonne vertébrale dont certaines vertèbres et plus particulièrement les vertèbres lombaires Va, Vb sont reliées entre elles par exemple par un dispositif d'ostéosynthèse rachidien 1 et par un dispositif de protection 10.

A titre d'exemple non limitatif le dispositif d'ostéosynthèse rachidien 1 est constitué d'éléments d'ancrage 2, 3 venant se fixer respectivement dans les pédicules Vp des vertèbres lombaires Va, Vb du segment rachidien Sr à instrumenter.

Le dispositif d'ostéosynthèse rachidien 1 comporte des éléments de liaison longitudinaux ou tige de liaison 4 disposés de part et d'autre des épineuses Ve des vertèbres lombaires Va, Vb afin de relier chaque premier élément d'ancrage 2 avec le second élément d'ancrage 3 correspondant.

Les premiers et seconds éléments d'ancrage 2, 3 comportent chacun une vis pédiculaire 5 et un connecteur de liaison 6 connus en soi.

Chaque vis pédiculaire 5 est constituée d'un corps longitudinal 50 présentant une partie filetée 51 destinée à l'ancrage de ladite vis dans le corps osseux des pédicules Vp de chaque vertèbre lombaire Va, Vb du segment rachidien Sr à instrumenter.

Le corps longitudinal 50 comprend dans le prolongement de la partie filetée 51 une tête 52 à profil sphérique permettant la mise en place et le réglage angulaire du connecteur de liaison 6.

Egalement, les vis pédiculaires 5 peuvent être canulées avec des orifices latéraux 53 permettant l'injection d'un ciment biologique destiné à améliorer l'ancrage dans chaque vertèbre lombaire Va, Vb du segment rachidien Sr à instrumenter.

Chaque connecteur de liaison 6 est constitué d'un corps 60 présentant un premier alésage 61 pour le logement de la tête sphérique 52 de la vis pédiculaire 5 correspondante. Le connecteur de liaison 6 est immobilisé sur la vis pédiculaire 5 dans une position angulaire déterminée au moyen d'un écrou de serrage 62 coopérant avec la partie supérieure filetée de l'alésage 61 afin de venir en appui serré contre la tête sphérique 52.

Le corps 60 de chaque connecteur de liaison 6 comporte, suivant une direction perpendiculaire à celle de l'alésage 61 et décalé latéralement, un autre alésage 63 permettant la mise en place de la tige de liaison 4 correspondante reliant lesdits connecteurs de liaison 6 entre eux.

Le corps 60 comprend suivant une direction parallèle à celle de l'alésage 61 un trou fileté 64 coopérant avec une vis de serrage 65 assurant l'immobilisation en translation et en rotation de la tige de liaison 4 par rapport audit connecteur 6.

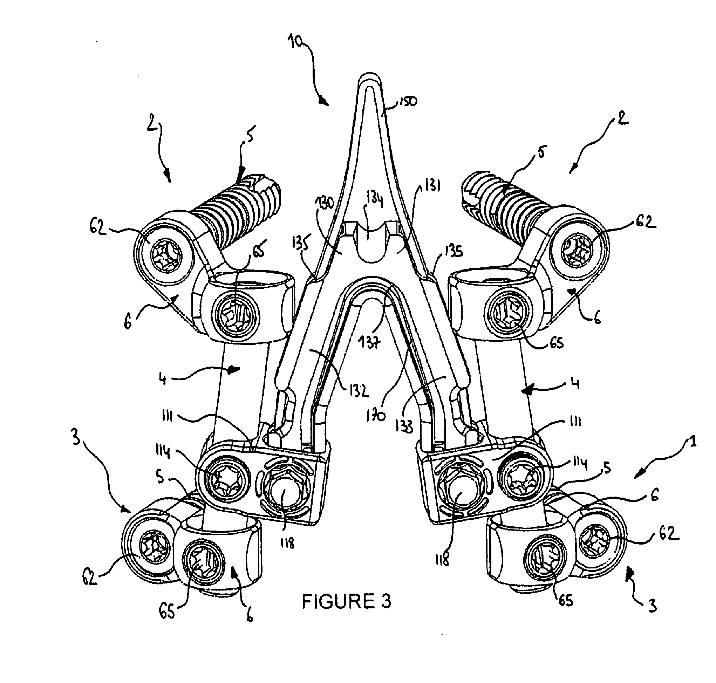

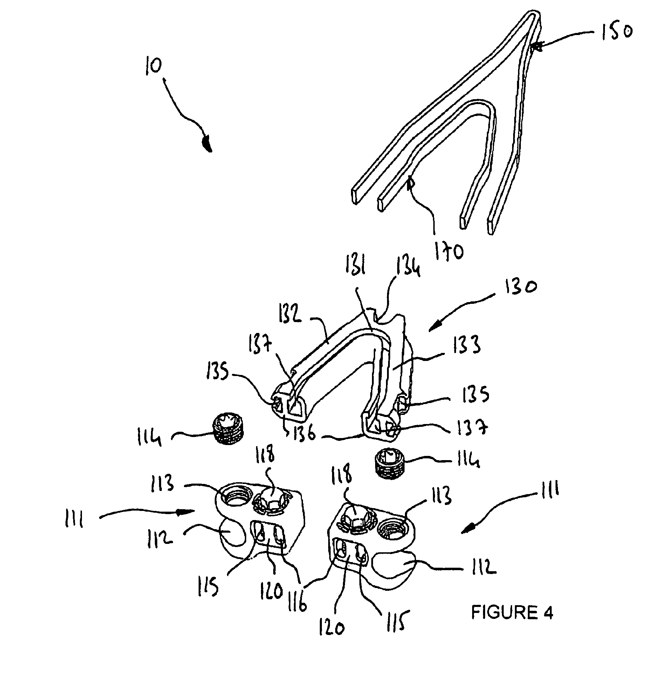

On a montré en figures 3 et 4 le dispositif de protection 10 suivant la présente invention qui est prévu pour venir s'agencer et se fixer sur n'importe quel dispositif d'ostéosynthèse rachidien 1 dès que ce dernier comprend, de part et d'autre des épineuses Ve des vertèbres Va, Vb instrumentés des tiges de liaison 4.

Le dispositif de protection 10 est constitué d'éléments de fixation 110 venant se positionner et se fixer sur chacune des tiges de liaison 4 du dispositif d'ostéosynthèse rachidien 1, d'une cale de soutien 130 venant prendre appui sur les éléments de fixation 110 et de ligaments synthétiques 150, 170 permettant de réunir ledit dispositif de protection 10 à l'épineuse Ve de la vertèbre sus-jacente Vc se trouvant juste au-dessus du segment rachidien Sr instrumenté par le dispositif d'ostéosynthèse rachidien 1.

L'élément de fixation 110 est constitué d'un connecteur 111 comportant un logement latéral 112 à profil interne cylindrique ouvert vers l'extérieur pour permettre l'encliquetage et l'introduction de la tige de liaison 4 correspondante du dispositif d'ostéosynthèse rachidien 1.

Le connecteur 111 est percé au-dessus du logement latéral 112 d'un alésage fileté 113 débouchant à l'intérieur dudit logement et coopérant avec une vis de serrage 114 permettant l'immobilisation en translation et en rotation de chaque élément de fixation 110 sur la tige de liaison 4 correspondante.

Le connecteur 111 comporte parallèlement à l'axe longitudinal du logement latéral 112 deux rainures 115, 116 de profil oblong traversant le connecteur 111 et permettant le passage des ligaments synthétiques 150, 170.

Les rainures 115, 116 débouchent à l'intérieur d'une cavité 117 ménagée au centre du connecteur 111 et à l'intérieur de laquelle coopère, suivant une direction perpendiculaire à celle des rainures, une came 118 permettant le serrage des ligaments synthétiques 150, 170.

Le connecteur 111 comporte, au niveau de chaque sortie des rainures 115,116, une empreinte 120 destinée à recevoir la cale de soutien 130 et dont le profil interne est semblable à celui externe de ladite cale de soutien.

La cale de soutien 130 du dispositif de protection 10 est réalisée dans un matériau élastique de manière à pouvoir se déformer sous une pression extérieure et plus particulièrement sous la pression de la vertèbre sus-jacente Vc.

La cale de soutien 130 présente un profil en forme de U renversé comprenant un pont supérieur 131 relié par deux jambes de soutien 132, 133 agencées de manière à passer de part et d'autre de l'épineuse Ve de la vertèbre Vb du segment rachidien Sr instrumenté.

Le pond supérieur 131 de la cale de soutien 130 comporte un logement 134 coopérant avec l'épineuse Ve de la vertèbre sus-jacente Vc.

Les jambes de soutien 132, 133 de la cale de soutien 130 comportent sur le profil externe du U renversé une première rainure 135 ouverte vers l'extérieur et permettant la mise en place, la retenue et le guidage du premier ligament synthétique 150.

La première rainure 135 est ménagée dans l'épaisseur et sur le côté de chaque jambe de soutien 132, 133 de la cale de soutien 130, afin que ladite rainure 135 débouche au niveau des pieds 136 desdites jambes de soutien 132, 133.

Les jambes de soutien 132, 133 et le pont supérieur 131 de la cale de soutien 130 comportent sur le profil interne du U renversé une seconde rainure 137 ouverte vers l'extérieur et permettant la mise en place, la retenue et le guidage du second ligament synthétique 170.

La seconde rainure 137 est ménagée de manière continue dans l'épaisseur de chaque jambe de soutien 132, 133 et du pont supérieur 131 de la cale de soutien 130 afin que ladite rainure 137 passe en dessous du logement 134 pour venir déboucher au niveau des pieds 136 desdites jambes de soutien 132, 133.

L'ouverture vers l'extérieur de la seconde rainure 137 est réalisée suivant une direction qui est perpendiculaire à celle prévue pour la première rainure 135.

Chaque ouverture vers l'extérieur de chaque rainure 135, 137 présente une dimension qui est inférieure à celle interne desdites rainures afin de constituer une retenue du ligament synthétique 150, 170 à l'intérieur de la rainure 135, 137 correspondante.

Le dispositif de protection 10 comporte deux ligaments synthétiques 150, 170 permettant de maintenir la cale de soutien 130 en forme de U renversé sur les éléments de fixation 110 fixés sur chacune des tiges de liaison 4 du dispositif d'ostéosynthèse rachidien 1.

Le premier ligament 150 logé à l'intérieur de la première rainure 135 de la cale de soutien 130 est prévu pour passer au-dessus de l'épineuse Ve de la vertèbre sus jacente Vc se trouvant juste au-dessus du segment rachidien Sr instrumenté afin que ladite épineuse Ve de la vertèbre sus-jacente Vc soit maintenue à l'intérieur du logement 134 de ladite cale, tandis que les deux extrémités libres dudit premier ligament 150 traversent respectivement les éléments de fixation 110 fixés sur chaque tige de liaison 4 par l'intermédiaire de la rainure 115 et de la came 118.

Le second ligament 170 logé à l'intérieur de la seconde rainure 137 de la cale de soutien 130 est prévu pour passer en dessous de l'épineuse Ve de la vertèbre sus-jacente Vc et au-dessus de l'épineuse Ve de la vertèbre Vb du segment rachidien Sr instrumenté, tandis que les deux extrémités libres dudit second ligament 170 traversent respectivement les éléments de fixation 110 fixés sur chaque tige de liaison 4 par l'intermédiaire de la rainure 116 et de la came 118.

On comprend aisément de la précédente description le fonctionnement du dispositif de protection 10 suivant la présente invention.

En effet, après la fusion des vertèbres Va et Vb du segment rachidien Sr par le dispositif d'ostéosynthèse rachidien 1, le chirurgien procède à la mise en place et la fixation du dispositif de protection 10 de l'étage sus-jacent formé par la vertèbre sus-jacente Vc.

Le chirurgien procède à la mise en place sur l'une des tiges de liaison 4 du dispositif d'ostéosynthèse rachidien 1, d'un élément de fixation 110 formé par un premier connecteur 111 qui est préalablement relié à la cale de soutien 130 par l'intermédiaire des deux ligaments synthétiques 150, 170 placés dans les rainures correspondantes 135, 137 de la jambe 132.

En effet, l'une des extrémités des deux ligaments synthétiques 150, 170 est retenue bloquée dans le premier connecteur 111 par l'intermédiaire de la came 118. La cale de soutien 130 est en appui contre le premier connecteur 111 afin que par exemple le pied de soutien 136 de la jambe 132 soit logé à l'intérieur de l'empreinte 120 dudit connecteur.

Le chirurgien positionne, par pivotement du premier connecteur 111 autour de la tige de liaison 4, la cale de soutien 130 pourvue des deux ligaments synthétiques 150,170 retenus dans le premier connecteur 111 entre la vertèbre Vb du segment rachidien Sr instrumenté par le dispositif d'ostéosynthèse rachidien 1 et la vertèbre sus-jacente Vc afin que l'épineuse Ve de ladite vertèbre sus-jacente Vc soit placée à l'intérieur du logement 134 ménagé dans le pont supérieur 131 de ladite cale de soutien 130.

Ensuite, le chirurgien dispose le ligament supérieur 150 autour de l'épineuse Ve de la vertèbre sus-jacente Vc et l'introduit dans la rainure 135 ménagée dans l'autre jambe 133 de la cale de soutien 130. Le chirurgien tend le ligament supérieur 150 et procède par l'intermédiaire de la vis de serrage 114 à l'immobilisation en translation et en rotation du premier connecteur 111 sur la tige de liaison 4 du dispositif d'ostéosynthèse rachidien 1.

Le chirurgien introduit les autres extrémités libres des ligaments supérieurs 150 et inférieurs 170 dans les rainures correspondantes 115 et 116 d'un autre élément de fixation 110 formé par un second connecteur 111 afin de traverser ce dernier.

Le chirurgien amène, tout en maintenant la tension sur les ligaments supérieurs 150 et inférieurs 170, le second connecteur 111 contre le pied de soutien 136 de la seconde jambe 133 de la cale de soutien 130, positionne ledit connecteur 111 sur l'autre tige de liaison 4 du dispositif d'ostéosynthèse rachidien 1 et l'immobilise en rotation et en translation par l'intermédiaire de la vis de serrage 114.

Le chirurgien procède, tout en maintenant la tension sur les ligaments supérieurs 150 et inférieurs 170, au serrage de ces derniers dans le second connecteur 111 au moyen de la came 118.

Enfin, le chirurgien coupe les extrémités des ligaments supérieurs 150 et inférieurs 170 dépassants des premiers et seconds connecteurs 111 du dispositif de protection 10.

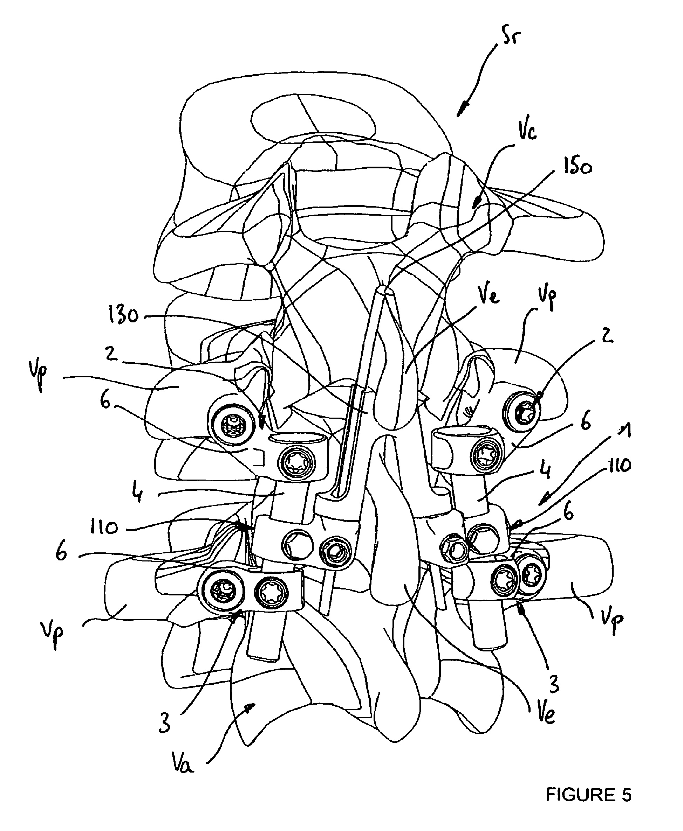

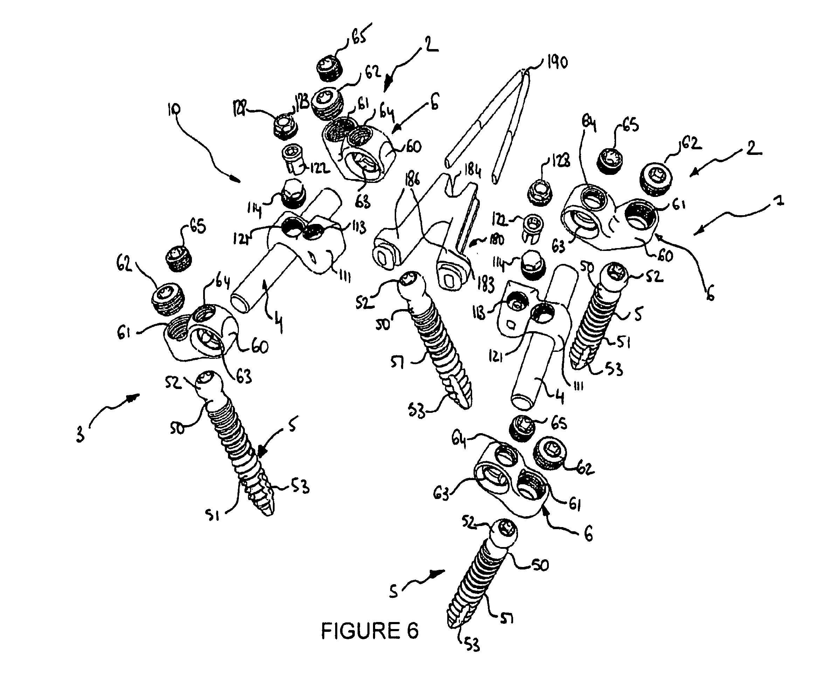

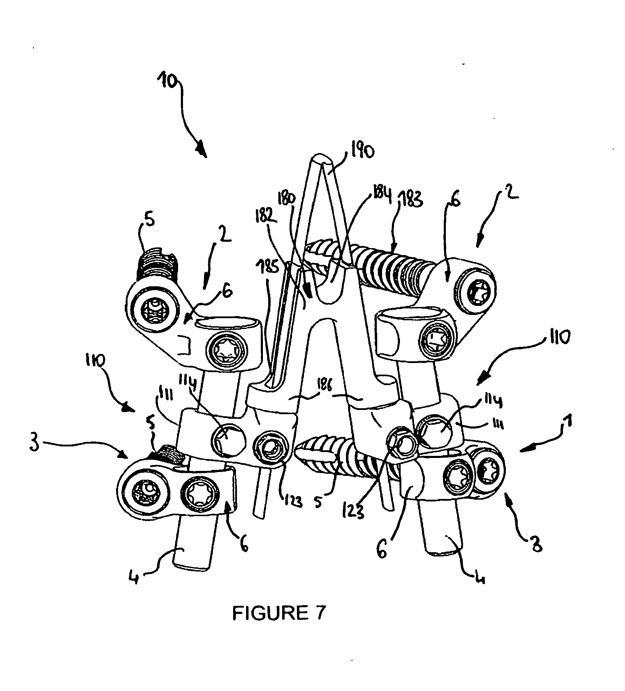

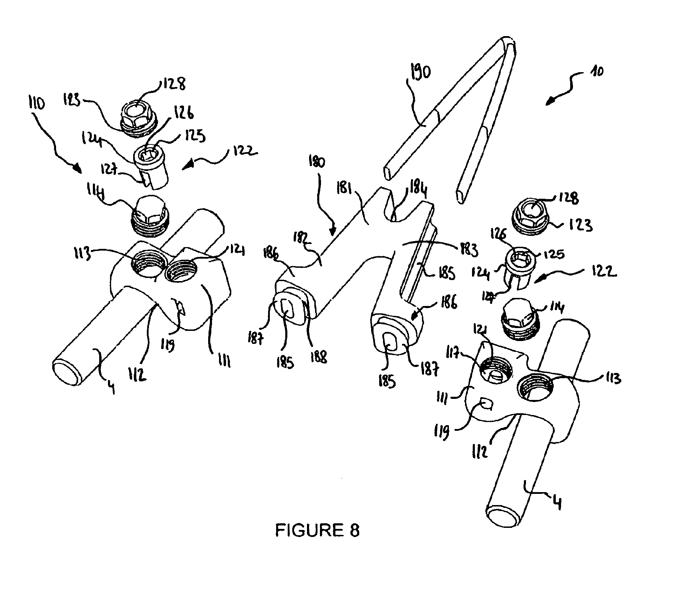

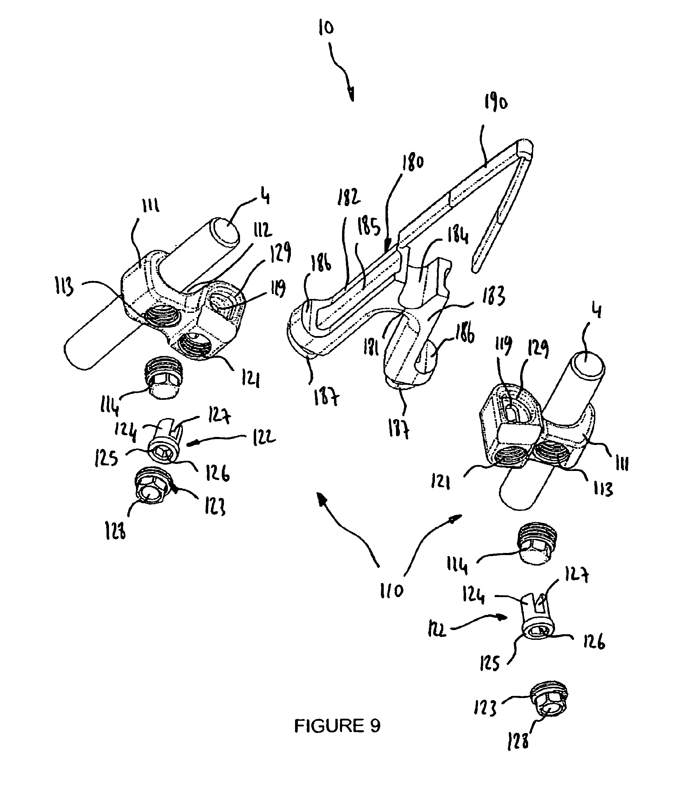

On a montré en figures 5 à 9 une variante du dispositif de protection 10 suivant la présente invention qui est prévu pour venir s'agencer et se fixer sur n'importe quel dispositif d'ostéosynthèse rachidien 1 dès que ce dernier comprend, de part et d'autre des épineuses Ve des vertèbres Va, Vb instrumentées des tiges de liaison 4.

Le dispositif d'ostéosynthèse rachidien 1 sur lequel est montée et fixée la variante du dispositif de protection 10 est semblable à celui décrit précédemment en figures 1 à 4 de la présente invention, mais se distingue principalement en ce qu'il comporte une cale de soutien 180 agencée pour ne recevoir qu'un seul ligament synthétique 190.

Le dispositif de protection 10 est constitué d'éléments de fixation 110 venant se positionner et se fixer sur chacune des tiges de liaison 4 du dispositif d'ostéosynthèse rachidien 1, d'une cale de soutien 180 venant prendre appui sur les éléments de fixation 110 et d'un ligament synthétique 190 permettant de réunir ledit dispositif de protection 10 à l'épineuse Ve de la vertèbre sus-jacente Vc se trouvant juste au-dessus du segment rachidien Sr instrumenté par le dispositif d'ostéosynthèse rachidien 1.

L'élément de fixation 110 est constitué d'un connecteur 111 comportant un logement latéral 112 à profil interne cylindrique ouvert vers l'extérieur pour permettre l'encliquetage et l'introduction de la tige de liaison 4 correspondante du dispositif d'ostéosynthèse rachidien 1.

Le connecteur 111 est percé au-dessus du logement latéral 112 d'un alésage fileté 113 débouchant à l'intérieur dudit logement et coopérant avec une vis de serrage 114 permettant l'immobilisation en translation et en rotation de chaque élément de fixation 110 sur la tige de liaison 4 correspondante.

Le connecteur 111 comporte parallèlement à l'axe longitudinal du logement latéral 112 une rainure 119 de profil oblong traversant le connecteur 111 et permettant le passage du ligament synthétique 190. La rainure 119 débouche à l'intérieur d'une cavité 117 ménagée au centre du connecteur 111 et à l'intérieur de laquelle débouche suivant une direction perpendiculaire à celle de la rainure un alésage fileté 121 permettant d'une part l'introduction d'une clef de verrouillage 122 à l'intérieur de la cavité 117 et d'autre par le serrage d'un contre-écrou 123.

La clef de verrouillage 122 est constituée d'un doigt cylindrique 124 comportant d'une part une tête 125 pourvue d'une empreinte 126 et d'autre part à l'opposé de ladite tête 125 et suivant un axe vertical et central une rainure 127 séparant ledit doigt cylindrique 124 en deux parties distinctes.

La rainure 127 est prévue pour être traversée par le ligament synthétique 190 lorsque la clef de verrouillage 122 est placée à l'intérieur de la cavité 117 du connecteur 111.

La clef de verrouillage 122 est immobilisée, après réglage en tension et immobilisation du ligament synthétique 190, à l'intérieur de la cavité 117 de chaque connecteur 111 par l'intermédiaire du contre écrou 123 qui vient après serrage en appui contre la tête 125.

Le contre écrou 123 comporte un alésage central et traversant 128 permettant le passage d'un outil afin que ce dernier puisse venir se loger à l'intérieur de l'empreinte 126 de la clef de verrouillage 122. L'outil permet d'entrainer en rotation la clef de verrouillage 122 à l'intérieur de la cavité 117 du connecteur correspondant 111 permettant, après mise en tension du ligament 190, de l'enrouler autour du doigt cylindrique 124 de ladite clef de verrouillage 122.

Chaque connecteur 111 comporte, au niveau de la rainure 119, une empreinte 129 destinée à recevoir chaque pied 186 de la cale de soutien 180 et dont le profil interne est semblable à celui externe de ladite cale de soutien.

La cale de soutien 180 du dispositif de protection 10 est réalisée dans un matériau élastique de manière à pouvoir se déformer sous une pression extérieure et plus particulièrement sous la pression de la vertèbre sus-jacente Vc.

La cale de soutien 180 présente un profil en forme de U renversé comprenant un pont supérieur 181 relié par deux jambes de soutien 182, 183 agencées de manière à passer de part et d'autre de l'épineuse Ve de la vertèbre Vb du segment rachidien Sr instrumenté.

Le pond supérieur 181 de la cale de soutien 180 comporte un logement 184 coopérant avec l'épineuse Ve de la vertèbre sus-jacente Vc.

Les jambes de soutien 182, 183 de la cale de soutien 180 comportent sur le profil externe du U renversé une rainure 185 ouverte vers l'extérieur et permettant la mise en place, la retenue et le guidage du ligament synthétique 190.

Chaque pied 186 se prolonge par une collerette 187 pourvue d'une gorge périphérique 188 permettant la retenue de la cale de soutien 180 à l'intérieur d'un logement de forme complémentaire réalisé à l'intérieur de chaque connecteur 111.

La rainure 185 traverse chaque pied 186 et la collerette 187 afin que cette dernière se trouve lors du montage de la cale 180 dans les connecteurs 111 dans le prolongement de la rainure 119 de ces derniers, pour que chaque connecteur soit traversé par l'extrémité libre du ligament 190.

On comprend aisément de la précédente description le fonctionnement du dispositif de protection 10 comportant un seul ligament 190.

En effet, après la fusion des vertèbres Va et Vb du segment rachidien Sr par le dispositif d'ostéosynthèse rachidien 1, le chirurgien procède à la mise en place et la fixation du dispositif de protection 10 de l'étage sus-jacent formé par la vertèbre sus-jacente Vc.

Le chirurgien procède à la mise en place sur l'une des tiges de liaison 4 du dispositif d'ostéosynthèse rachidien 1, d'un élément de fixation 110 formé par un premier connecteur 111 préalablement relié à la cale de soutien 180 et au ligament synthétique 190.

En effet, la collerette 187 du pied 186 de la cale de soutien 180 est surmoulée dans le logement 129 prévu à cet effet du connecteur 111, tandis que l'une des extrémités libres du ligament 190 est bloquée dans ledit connecteur au moyen de la clef de verrouillage 122 et du contre-écrou 123.

Le chirurgien positionne, par pivotement du premier connecteur 111 autour de la tige de liaison 4, la cale de soutien 180 pourvue du ligament synthétique 190 retenu dans le premier connecteur 111 entre la vertèbre Vb du segment rachidien Sr instrumenté par le dispositif d'ostéosynthèse rachidien 1 et la vertèbre sus-jacente Vc afin que l'épineuse Ve de ladite vertèbre sus-jacente Vc soit placée à l'intérieur du logement 184 ménagé dans le pont supérieur 181 de ladite cale de soutien 180.

Ensuite, le chirurgien dispose le ligament 190 autour de l'épineuse Ve de la vertèbre sus-jacente Vc et l'introduit dans la rainure 185 ménagée dans les jambes 182, 183 de la cale de soutien 180. Le chirurgien procède par l'intermédiaire de la vis de serrage 114 à l'immobilisation en translation et en rotation du premier connecteur 111 sur la première tige de liaison 4 du dispositif d'ostéosynthèse rachidien 1.

Le chirurgien introduit l'autre extrémité libre du ligament 190 dans la rainure correspondante 119 d'un autre élément de fixation 110 formé par un second connecteur 111 afin de traverser ce dernier. Le connecteur 111 est monté sur l'autre tige de liaison 4 du dispositif d'ostéosynthèse rachidien 1 afin de traverser ce dernier.

Le chirurgien enfile l'ensemble cale 180 et connecteur 110 entre les épineuses Ve puis positionne par pivotement du premier connecteur 111 autour de la tige de liaison 4 puis du second connecteur puis sert les deux vis 114.

Le chirurgien procède d'une part à la mise en tension du ligament 190, et d'autre part à l'immobilisation en rotation et en translation du second connecteur 111 par l'intermédiaire de la vis de serrage 114 sur l'autre tige de liaison 4 du dispositif d'ostéosynthèse rachidien 1.

Le chirurgien procède, tout en maintenant la tension du ligament 190 au blocage de ce dernier par l'entrainement en rotation de la clef de verrouillage 122 à l'intérieur de la cavité 117 afin que le ligament 190 s'enroule autour du doigt cylindrique 124.

Le chirurgien maintient la clef de verrouillage 122 en position pendant son blocage en rotation par le contre-écrou 123.

Enfin, le chirurgien coupe les extrémités du ligament 190 dépassant des premiers et seconds connecteurs 111 du dispositif de protection 10.

Il doit d'ailleurs être entendu que la description qui précède n'a été donnée qu'à titre d'exemple, et qu'elle ne limite nullement le domaine de l'invention dont on ne sortirait pas en remplaçant les détails d'exécution décrits par tout autre équivalent.

## Revendications

1. Dispositif de protection des étages adjacents Vc d'un segment rachidien Sr d'une colonne vertébrale instrumentée par un dispositif d'ostéosynthèse (1) comprenant des tiges de liaison (4), ledit dispositif de protection, étant constitué des éléments de fixation (110) formés de connecteurs (111) capables d'être immobilisés en translation et en rotation sur la tige de liaison (4) correspondante, d'une cale de soutien, présentant un profil en forme de U renversé et réalisée dans un matériau élastique dans laquelle est logée l'épineuse Ve de la vertèbre sus-jacente Vc se trouvant juste au-dessus du segment rachidien Sr instrumenté, d'au moins un ligament (150, 170 ; 190) permettant de réunir l'épineuse Ve de la vertèbre sus-jacente Vc à la cale de soutien (130 ; 180) et la cale de soutien (130 ; 180) aux connecteurs (111) et des moyens de blocage (118, 122) du ligament (150, 170; correspondant, après sa mise en tension, dans les connecteurs (111), **caractérisé en ce que** les connecteurs (111) comportent des rainures (115, 116 ; 119) débouchant à l'intérieur d'une cavité (117) et permettant le passage du ligament et d'une empreinte ou logement (120 ;129) destinée à recevoir ladite cale de soutien (130 ; 180) et **en ce que** lesdits moyens de blocage sont constitués d'une came (118) ou d'une clef de verrouillage (122) logé à l'intérieur de ladite cavité (117) suivant une direction perpendiculaire à celle desdites rainures (115, 116 ; 119) et permettant le serrage du ligament correspondant (150, 170 ; 190).

2. Dispositif de protection suivant la revendication 1, **caractérisé en ce que** la cale de soutien (130) présente un profil en forme de U renversé comprenant un pont supérieur (131) se prolongeant par des jambes de soutien (132, 133) dans lesquelles sont ménagées des rainures (135, 137) pour permettre respectivement la mise en place et la retenue des ligaments, supérieur (150) et inférieur (170).

3. Dispositif de protection suivant la revendication 2, **caractérisé en ce que** la première rainure (135) est ouverte vers l'extérieur et ménagée sur le côté de chaque jambe de soutien (132, 133) de la cale de soutien (130).

4. Dispositif de protection suivant la revendication 2, **caractérisé en ce que** la seconde rainure (137) est ouverte vers l'extérieur et ménagée de manière continue dans l'épaisseur de chaque jambe de soutien (132, 133) et du pont supérieur (131) de la cale de soutien (130).

5. Dispositif de protection suivant la revendication 2, **caractérisé en ce que** le pont supérieur (131) comporte un logement (134) permettant la mise en place de l'épineuse Ve de la vertèbre sus-jacente Vc.

6. Dispositif de protection suivant les revendications 3 et 4, **caractérisé en ce que** la rainure (137) passe en dessous du logement (134) ménagé dans le pont (131).

7. Dispositif de protection suivant la revendication 4, **caractérisé en ce que** l'ouverture vers l'extérieur de la seconde rainure (137) est réalisée suivant une direction qui est perpendiculaire à celle prévue pour la première rainure (135).

8. Dispositif de protection suivant la revendication 1, **caractérisé en ce que** chaque connecteur (111) comporte un logement latéral (112) à profil interne cylindrique permettant l'encliquetage et l'introduction de la tige de liaison (4) correspondante, un alésage fileté (113) débouchant à l'intérieur dudit logement et coopérant avec une vis de serrage (114) permettant l'immobilisation en translation et en rotation de chaque élément de fixation (110), une rainure (119) de profil oblong traversant le connecteur (111) et permettant le passage du ligament synthétique (190), une cavité (117) ménagée au centre du connecteur (111) et à l'intérieur de laquelle débouche suivant une direction perpendiculaire à celle de la rainure (119) un alésage fileté (121) permettant d'une part l'introduction d'une clef de verrouillage (122) à l'intérieur de ladite cavité (117) et d'autre par le serrage d'un contre-écrou (123) permettant d'immobiliser en rotation ladite clef de verrouillage (122).

9. Dispositif de protection suivant la revendication 8, **caractérisé en ce que** la clef de verrouillage (122) est constituée d'un doigt cylindrique (124) comportant d'une part une tête (125) pourvue d'une empreinte (126) et d'autre part à l'opposé de ladite tête (125) et suivant un axe vertical et central une rainure (127) séparant ledit doigt cylindrique (124) en deux parties distinctes, ladite rainure (127) étant prévue pour être traversée par le ligament synthétique (190) lorsque la clef de verrouillage (122) est placée à l'intérieur de la cavité (117) du connecteur (111).

10. Dispositif de protection suivant la revendication 8, **caractérisé en ce que** le contre écrou (123) comporte un alésage central et traversant (128) pour le passage d'un outil afin que ce dernier puisse venir se loger à l'intérieur de l'empreinte (126) de la clef de verrouillage (122) en vue d'entrainer en rotation ladite clef de verrouillage (122) à l'intérieur de la cavité (117) du connecteur correspondant (111) pour l'enroulement du ligament (190) autour du doigt cylindrique (124).

11. Dispositif de protection suivant la revendication 1, **caractérisé en ce que** la cale de soutien (180) présente un profil en forme de U renversé comprenant un pont supérieur (181) relié par deux jambes de soutien (182, 183) agencées de manière à passer de part et d'autre de l'épineuse Ve de la vertèbre Vb du segment rachidien Sr instrumenté, ledit pond supérieur (181) comportant un logement (184) coopérant avec l'épineuse Ve de la vertèbre sus-jacente Vc, tandis que lesdites jambes de soutien (182, 183) comportent d'une part sur le profil externe du U renversé une rainure (185) ouverte vers l'extérieur et permettant la mise en place, la retenue et le guidage du ligament synthétique (190) et d'autre part à chaque extrémité libre un pied (186) se prolongeant par une collerette (187) pourvue d'une gorge périphérique (188) permettant la retenue de la cale de soutien (180) à l'intérieur d'un logement (129) de forme complémentaire réalisé à l'intérieur de chaque connecteur (111).

12. Dispositif de protection suivant la revendication 15, **caractérisé en ce que** la rainure (185) traverse chaque pied (186) et la collerette (187) afin que cette dernière se trouve lors du montage de la cale (180) dans les connecteurs (111) dans le prolongement de la rainure (119).

## Patentansprüche

1. Schutzvorrichtung der angrenzenden Ebenen Vc eines spinalen Segments Sr einer von einer Osteosynthesevorrichtung (1) instrumentierten Wirbelsäule, die Verbindungsstangen (4) umfasst, wobei die Schutzvorrichtung von Befestigungselementen (110) gebildet wird, die von Verbindern (111) gebildet werden, die imstande sind, auf der entsprechenden Verbindungsstange (4) eines Stützkeils in Verschiebung und in Rotation blockiert zu sein, der ein Profil in Form eines umgekehrten Us aufweist und aus einem elastischen Material hergestellt ist, in dem der Dorn Ve des darüberliegenden Wirbels Vc ruht, der sich direkt über dem instrumentierten spinalen Segments Sr befindet, wobei mindestens ein Band (150, 170; 190) erlaubt, den Dorn Ve des darüberliegenden Wirbels Vc mit dem Stützkeil (130; 180) zu verbinden und den Stützkeil (130; 180) mit den Verbindern (111) und Blockiermitteln (118, 122) des entsprechenden Bands (150, 170; 190) nach seiner Verspannung in den Verbindern (111), **dadurch gekennzeichnet, dass** die Verbinder (111) Rillen (115, 116; 119) aufweisen, die in einen Hohlraum (117) ausmünden und den Durchgang des Bands erlauben, und einen Eindruck oder eine Aufnahme (120; 129), der dazu vorgesehen sind, den Stützkeil (130; 180) aufzunehmen und dass die Blockiermittel von einem Keil (118) oder einem Verriegelungsschlüssel (122) gebildet werden, der in dem Hohlraum (117) gemäß einer Richtung lotrecht zur Richtung der Rillen (115, 116; 119) ruht und die Verspannung des entsprechenden Bands (150, 170; 190) erlaubt.

2. Schutzvorrichtung nach Anspruch 1, **dadurch gekennzeichnet, dass** der Stützkeil (130) ein Profil in Form eines umgekehrten Us aufweist, das eine obere Brücke (131) umfasst, die sich anhand von Stützschenkeln (132, 133) verlängert, in die Rillen (135, 137) eingearbeitet sind, um das Platzieren und das Halten der oberen (150) und unteren (170) Bands zu erlauben.

3. Schutzvorrichtung nach Anspruch 2, **dadurch gekennzeichnet, dass** die erste Rille (135) nach außen geöffnet ist und auf der Seite jedes Stützschenkels (132, 133) des Stützkeils (130) eingearbeitet ist.

4. Schutzvorrichtung nach Anspruch 2, **dadurch gekennzeichnet, dass** die zweite Rille (137) nach außen geöffnet ist und kontinuierlich in die Dicke jedes Stützschenkels (132, 133) und der oberen Brücke (131) des Stützkeils (130) eingearbeitet ist.

5. Schutzvorrichtung nach Anspruch 2, **dadurch gekennzeichnet, dass** die obere Brücke (131) eine Aufnahme (134) aufweist, die die Platzierung des Dorns Ve des darüberliegenden Wirbels Vc erlaubt.

6. Schutzvorrichtung nach den Ansprüchen 3 und 4, **dadurch gekennzeichnet, dass** die Rille (137) unter der in die Brücke (131) eingearbeitete Aufnahme (134) verläuft.

7. Schutzvorrichtung nach Anspruch 4, **dadurch gekennzeichnet, dass** die Öffnung nach außen der zweiten Rille (137) gemäß einer Richtung durchgeführt ist, die lotrecht zu der Richtung ist, die für die erste Rille (135) vorgesehen ist.

8. Schutzvorrichtung nach Anspruch 1, **dadurch gekennzeichnet, dass** jeder Verbinder (111) eine seitliche Aufnahme (112) mit zylindrischem Innenprofil aufweist, die das Rasten und das Einführen der entsprechenden Verbindungsstange (4) erlaubt, wobei eine Gewindebohrung (113) in die Aufnahme ausmündet und mit einer Spannschraube (114) zusammenarbeitet, die die Blockade in Verschiebung und in Rotation jedes Befestigungselements (110) erlaubt, wobei eine Rille (119) mit länglichem Profil den Verbinder (111) durchquert und den Durchgang des synthetischen Bands (190) erlaubt, wobei ein Hohlraum (117), der im Zentrum des Verbinders (111) ausgebildet ist und in den gemäß einer Richtung lotrecht zur Richtung der Rille (119) eine Gewindebohrung (121) ausmündet, einerseits das Einführen eines Verriegelungsschlüssels (122) in den Hohlraum (117) und andererseits das Verspannen einer Gegenmutter (123) erlaubt, wodurch erlaubt wird, den Verriegelungsschlüssel (122) in Rotation zu blockieren.

9. Schutzvorrichtung nach Anspruch 8, **dadurch gekennzeichnet, dass** der Verriegelungsschlüssel (122) aus einem zylindrischen Finger (124) besteht, der auf der einen Seite einen Kopf (125) mit einem Eindruck (126) und auf der anderen Seite gegenüber dem Kopf (125) und gemäß einer vertikalen und zentralen Achse eine Rille (127) aufweist, die den zylindrischen Finger (124) in zwei unterschiedliche Teile teilt, wobei die Rille (127) dazu bestimmt ist, von dem synthetischen Band (190) durchquert zu werden, wenn der Verriegelungsschlüssel (122) im Hohlraum (117) des Verbinders (111) platziert ist.

10. Schutzvorrichtung nach Anspruch 8, **dadurch gekennzeichnet, dass** die Gegenmutter (123) eine zentrale und durchgängige Bohrung (128) für den Durchgang eines Werkzeugs aufweist, damit dieses im Eindruck (126) des Verriegelungsschlüssels (122) ruhen kann, um den Verriegelungsschlüssel (122) im Hohlraum (117) des entsprechenden Verbinders (111) für das Aufrollen des Bands (190) um den zylindrischen Finger (124) in Rotation anzutreiben.

11. Schutzvorrichtung nach Anspruch 1, **dadurch gekennzeichnet, dass** der Stützkeil (180) ein Profil in Form eines umgekehrten Us aufweist, das eine obere Brücke (181) umfasst, die durch zwei Stützschenkel (182, 183) verbunden ist, die derart ausgebildet sind, dass sie auf der einen und der anderen Seite des Dorns Ve des Wirbels Vb des instrumentierten spinalen Segments Sr verlaufen, wobei die obere Brücke (181) eine Aufnahme (184) aufweist, die mit dem Dorn Ve des darüberliegenden Wirbels Vc zusammenarbeitet, wogegen die Stützschenkel (182, 183) einerseits auf dem externen Profil des umgekehrten Us eine nach außen geöffnete Rille (185) aufweisen, die das Platzieren, das Halten und das Führen des synthetischen Bands (190) erlaubt und andererseits an jedem freien Ende einen Fuß (186), der durch einen Ring (187) mit einer Umfangsnut (188) verlängert wird, der erlaubt, den Stützkeil (180) in einer komplementären Aufnahme (129) komplementärer Form zu halten, die in jeden Verbinder (111) eingearbeitet ist.

12. Schutzvorrichtung nach Anspruch 15, **dadurch gekennzeichnet, dass** die Rille (185) jeden Fuß (186) und den Ring (187) durchquert, damit sich dieser bei der Montage des Keils (180) in den Verbindern (111) in Verlängerung der Rille (119) befindet.

## Claims

1. A device for protecting adjacent levels Vc of a spinal segment Sr of a vertebral column instrumented by an osteosynthesis device (1) comprising connecting rods (4), said protection device being made up of fastening elements (110) formed by connectors (111) capable of being translationally and rotationally immobilized on the corresponding connecting rod (4) of a support wedge, **characterized in that** the connectors (111) comprise slots (115, 116; 119) emerging inside a recess (117) and allowing the passage of the ligament, and a cavity or housing (120; 129) intended to receive said support wedge (130; 180) having an upside down U-shaped profile and made from an elastic material in which the spine Ve of the superjacent vertebra Vc located just above the instrumented spinal segment Sr is housed, at least one ligament (150, 170; 190) making it possible to join the spine Ve of the superjacent vertebra Vc to the support wedge (130; 180) and the support wedge (130; 180) to the connectors (111) and the locking means (118, 122) of the corresponding ligament (150, 170; 190), after tensioning thereof, in the connectors (111), and **in that** said locking means are made up of a cam (118) or a locking key (122) housed inside said recess (117) in a direction perpendicular to that of the slots (115, 116; 119) and allowing gripping of the corresponding ligament (150, 170; 190).

2. The protection device according to claim 1, **characterized in that** the support wedge (130) has a profile in the shape of an upside down U comprising an upper bridge (131) extending via support links (132, 133) in which slots (135, 137) are formed to respectively allow the placement and retention of the upper (150) and lower (170) ligaments.

3. The protection device according to claim 2, **characterized in that** the first slot (135) is open toward the outside and formed on the side of each support leg (132, 133) of the support wedge (130).

4. The protection device according to claim 2, **characterized in that** the second slot (137) is open toward the outside and formed continuously in the thickness of each support leg (132, 133) and the upper bridge (131) of the support wedge (130).

5. The protection device according to claim 2, **characterized in that** the upper bridge (131) includes a housing (134) allowing the placement of the spine Ve of the superjacent vertebra Vc.

6. The protection device according to claims 3 and 4, **characterized in that** the slot (137) passes below the housing (134) formed in the bridge (131).

7. The protection device according to claim 4, **characterized in that** the outward opening of the second slot (137) is done in a direction that is perpendicular to that provided for the first slot (135).

8. The protection device according to claim 1, **characterized in that** each connector (111) includes a side housing (112) with a cylindrical inner profile allowing the snapping and insertion of the corresponding connecting rod (4), a threaded bore (113) emerging inside said housing and cooperating with the tightening screw (114) allowing the translational and rotational immobilization of each fastening element (110), a slot (119) with an oblong profile passing through the connector (111) and allowing the passage of the synthetic ligament (190), a recess (117) formed in the center of the connector (111) and inside which a threaded bore (121) emerges, in a direction perpendicular to that of the slot (119), on the one hand allowing the locking key (122) to be inserted inside said recess (117) and on the other hand allowing a locknut (123) to be tightened, making it possible to immobilize the rotation of said locking key (122).

9. The protection device according to claim 8, **characterized in that** the locking key (122) is made up of a cylindrical finger (124) including a head (125) provided with a cavity (126) on the one hand, and opposite said head (125) and along a vertical central axis, a slot (127) separating said cylindrical finger (124) into two separate parts on the other hand, said slot (127) being provided to be crossed through by the synthetic ligament (190) when the locking key (122) is placed inside the recess (117) of the connector (111).

10. The protection device according to claim 8, **characterized in that** the locknut (123) includes a central through bore (128) for the passage of a tool so that the latter can be housed inside the cavity (126) of the locking key (122) so as to rotate said locking key (122) inside the recess (117) of the corresponding connector (111) to wind the ligament (190) around the cylindrical finger (124).

11. The protection device according to claim 1, **characterized in that** the support wedge (180) has a profile in the shape of an upside down U comprising an upper bridge (181) connected by two support legs (182, 183) arranged so as to pass on either side of the spine Ve of the vertebra Vb of the instrumented spinal segment Sr, said upper bridge (181) including a housing (24) cooperating with the spine Ve of the superjacent vertebra Vc, while said support legs (182, 183) include, on the one hand, on the outer profile of the upside down U, an outwardly open slot (185) allowing the placement, retention and guiding of the synthetic ligament (190), and, on the other hand, at each free end, a foot (26) extending via a flange (187) provided with a peripheral groove (188) allowing the retention of the support wedge (180) inside a housing (129) with a complementary shape produced inside each connector (111).

12. The protection device according to claim 15, **characterized in that** the slot (185) passes through each foot (186) and the flange (187) so that the latter, during mounting of the wedge (180) in the connectors (111), is in the extension of the slot (119).
